# EUROPÄISCHE PATENTANMELDUNG

(11) **EP 1 800 631 A2**
(43) Veröffentlichungstag der Anmeldung: **27.06.2007**
(21) Anmeldenummer: 06405243.4
(22) Anmeldetag: 31.05.2006
(51) Int. Cl.: A61F 5/34, A61F 5/56, A47G 9/10

(54) **Orthopädisches Luft/-Wasserkissen**

(30) Priorität: 15.06.2005 CH 10142005
(71) Anmelder: CH-Medical GmbH, 6645 Brione sopra Minusio (CH)
(72) Erfinder: Zeindler, Kurt, 6030 Ebikon (CH)

(57) **Zusammenfassung**

Die Erfindung betrifft ein Orthopädisches Schlaf-Ruhe-Kissen mit einer Schicht Polyestermaterial und/oder Memory Foam und zusätzlich je 1 Wasserkammer und 1 Luftkammer.

Herkömmliche Kissen haben die Eigenschaft, dass der darauf gelagerte Kopf - sich auf die Basis des Füllmaterials zusammenschrumpft und somit Schuftereinbuchtungen und ein verkrampfter Körper resultieren. Eine richtig orthopädische Körperlinie kann nicht gewährleistet werden. Ebenfalls ist keine Stützung des Halswirbels vorhanden. Daraus resultierende Kopfschmerzen, Rückenschmerzen, Verkrampfungen des Rückens bringen oft lang und kostspielige Behandlungen beim Orthopaden oder Physio-Therapeuten mit sich.

Es war nun die Aufgabe ein orthopädisch - richtiges Schlaf-Ruhe-Kissen zu entwickein, das die Vorteile eines antiallergischen Inhaltes, welcher Kontakt zum Kopf hat, sowie sich durch unterschiedliche Mengen von Wasser in einer Wasserkammer sich dem Körper (Schulter für seitwärts schlafen) angepasst werden kann. (wenig Wasser für kleinere Personen -viel Wasser für grossschulterige Personen). Das eingefüllte Wasser ergibt gleichzeitig ein bestimmtes Gewicht des Kissens und gilt somit als fixer Standort im Bett oder Liege. Da ältere Personen auch Probleme haben Gewichte zu verschieben oder handzuhaben und ein "nur" mit Wasser gefülltes Ein-Kammer-System zuwenig Stabilität hat - haben wir eine zusätzliche Kammer geschaffen, die als Unterlage mit Luft gefüllt werden kann. Eine "einstellbare" Stabilität wird damit geschaffen und die Kombination mit Wasser ergibt eine optimale - orthopädisch richtige Kopfauflage.

Ein Schutzanzug kann wie bei den üblichen Kissen angezogen werden. Eine Handwäsche gewährleistet die gewünschte Sauberkeit des Kissens. Die Materialien sind so gewählt worden, dass eine Lebensdauer bis zu 5 Jahren bei richtiger Handhabung gerechnet werden kann.

## Beschreibung

Die Erfindung betrifft ein Orthopädisches Schlaf-Ruhe-Kissen mit einer Schicht Polyestermaterial und/oder Memory Foam und zusätzlich je 1 Wasserkammer und 1 Luftkammer.

Herkömmliche Kissen haben die Eigenschaft, dass der darauf gelagerte Kopf - sich auf die Basis des Füllmaterials zusammenschrumpft und somit Schultereinbuchtungen und ein verkrampfter Körper resultieren. Eine richtig orthopädische Körperlinie kann nicht gewährleistet werden. Ebenfalls ist keine Stützung des Halswirbels vorhanden. Daraus resultierende Kopfschmerzen, Rückenschmerzen, Verkrampfungen des Rückens bringen oft lang und kostspielige Behandlungen beim Orthopäden oder Physio-Therapeuten mit sich.

Es war nun die Aufgabe ein orthopädisch - richtiges Schlaf-Ruhe-Kissen zu entwickeln, das die Vorteile eines antiallergischen Inhaltes, welcher Kontakt zum Kopf hat, sowie sich durch unterschiedliche Mengen von Wasser in einer Wasserkammer sich dem Körper (Schulter für seitwärts schlafen) angepasst werden kann. (wenig Wasser für kleinere Personen - viel Wasser für grossschulterige Personen). Das eingefüllte Wasser ergibt gleichzeitig ein bestimmtes Gewicht des Kissens und gilt somit als fixer Standort im Bett oder Liege. Da ältere Personen auch Probleme haben Gewichte zu verschieben oder handzuhaben und ein "nur" mit Wasser gefülltes Ein-Kammer-System zuwenig Stabilität hat - haben wir eine zusätzliche Kammer geschaffen, die als Unterlage mit Luft gefüllt werden kann. Eine "einstellbare" Stabilität wird damit geschaffen und die Kombination mit Wasser ergibt eine optimale - orthopädisch richtige Kopfauflage.

Ein Schutzanzug kann wie bei den üblichen Kissen angezogen werden. Eine Handwäsche gewährleistet die gewünschte Sauberkeit des Kissens. Die Materialien sind so gewählt worden, dass eine Lebensdauer bis zu 5 Jahren bei richtiger Handhabung gerechnet werden kann.

### Zeichnung des Luft-/Wasserkissens

Es zeigen:
- Fig. 1: ein Schnitt durch das Kissen (70x40cm) mit der Polyesterschicht, Wasserkammer, Luftkammer
- Fig. 2: Einfüll-Stutzen für das Wasser / Einfüll-Ventil für die Luft
- Fig. 3: - wenig Wasser gefüllt mit viel Luft
- Fig. 4: - viel Wasser gefüllt mit wenig Luft
- Fig. 5: - ½ Wasser gefüllt mit ½ Luft

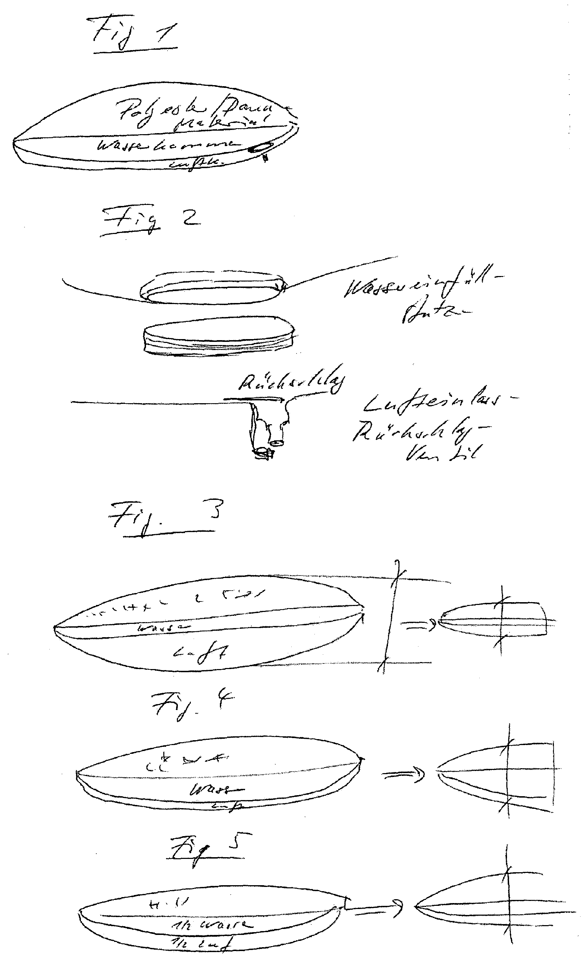

Diese Figuren zeigen die Vielfältigkeit der Anwendung des Wasser-Luft-Kissens. Eine orthopädisch richtig dosierte Kopfauflage ist damit gewährleistet.

## Patentansprüche

1. Orthopädisches Wasser-Luft-Kissen mit einer Kammer Polyester/und/oder Daunenmaterial sowie einer Wasserkammer mit sep. Einfüllstutzen sowie mit einer Luftkammer mit sep. Lufteinlass-Ventil/Rückschlagventil.

2. Orthopädisches Wasser-Luft-Kissen nach Patentanspruch **dadurch gekennzeichnet, dass** die erste Kammer gefüllt mit Polyester/und/oder Daunenmaterial separat als direkte Kopfauflage gebraucht wird.

3. Orthopädisches Wasser-Luft-Kissen nach Patentanspruch **dadurch gekennzeichnet, dass** die zweite Kammer gefüllt mit Wasser von 1- 5 Liter durch einen sep. Wassereinfüllstutzen als eine einzige sep. Kammer gefüllt werden kann.

4. Orthopädisches Wasser-Luft-Kissen nach Patentanspruch **dadurch gekennzeichnet, dass** die dritte Kammer gefüllt mit Luft von 1-5 Liter durch ein sep. Luft/Rückschlagventil als einzige sep. Kammer gefüllt werden kann.

5. Orthopädisches Wasser-Luft-Kissen nach Patentanspruch **dadurch gekennzeichnet, dass** alle 3 Kammern kombinierte Füllungen sowie dem Körper angepasste Füllmengen gleichzeitig gebraucht werden kann.

6. Orthopädisches Wasser-Luft-Kissen nach Patentanspruch in einer rechteckigen Grösse von 50x70cm sowie 30x40cm. mit drei sep. Kammern und Einfüllstutzen sowie Luft/Rückschlagventil.
